(19) Europäisches Patentamt
European Patent Office
Office européen des brevets

(11) EP 1 140 794 B1

(12) # FASCICULE DE BREVET EUROPEEN

(45) Date de publication et mention de la délivrance du brevet: **21.01.2004 Bulletin 2004/04**

(21) Numéro de dépôt: **00900532.3**

(22) Date de dépôt: **07.01.2000**

(51) Int Cl.[7]: **C07C 227/32**, C07C 227/24, C07D 233/84

(86) Numéro de dépôt international: **PCT/FR2000/000020**

(87) Numéro de publication internationale: **WO 2000/040545 (13.07.2000 Gazette 2000/28)**

(54) **NOUVEAU PROCEDE DE PREPARATION D'AMINOACIDES CHIRAUX**

VERFAHREN ZUR HERSTELLUNG VON CHIRALEN AMINOSÄUREN

NOVEL METHOD FOR PREPARING CHIRAL AMINO ACIDS

(84) Etats contractants désignés: **AT BE CH CY DE DK ES FI FR GB GR IE IT LI LU MC NL PT SE**

(30) Priorité: **07.01.1999 FR 9900202**

(43) Date de publication de la demande: **10.10.2001 Bulletin 2001/41**

(73) Titulaire: **Bayer CropScience S.A. 69009 Lyon (FR)**

(72) Inventeur: **PELTA, Isabelle F-69680 Chassieu (FR)**

(56) Documents cités:
**EP-A- 0 629 616**
**WO-A-98/03490**
**FR-A- 2 499 560**

- **PATENT ABSTRACTS OF JAPAN vol. 011, no. 317 (C-452), 15 octobre 1987 (1987-10-15) & JP 62 103049 A (MITSUI TOATSU CHEM INC), 13 mai 1987 (1987-05-13) cité dans la demande**
- **PATENT ABSTRACTS OF JAPAN vol. 010, no. 094 (C-338), 11 avril 1986 (1986-04-11) & JP 60 224661 A (MITSUI TOATSU KAGAKU KK), 9 novembre 1985 (1985-11-09) cité dans la demande**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 140 794 B1

Printed by Jouve, 75001 PARIS (FR)

## Description

### Domaine de l'invention

La présente invention concerne un nouveau procédé de préparation d'aminoacides chiraux à partir d'hydantoïnes racémiques et l'utilisation des dits aminoacides chiraux en tant qu'intermédiaires de synthèse de composés organiques chiraux.

### État de la technique

Il existe de nombreux procédés de préparation d'aminoacides chiraux dans la littérature, principalement basés sur des procédés de résolution de mélange racémiques par chromatographie liquide, résolution au moyen d'alcaloïdes ou par procédé enzymatique, comme par exemple dans la demande de brevet français non encore publiée n° 98 06339.

Ces différents procédés de résolution offrent les désavantages d'être des procédés difficilement industrialisables et d'un coût relativement élevé. En effet, les méthodes utilisées sont coûteuses.

Les procédés industriels - on entend par procédés industriels tous procédés de préparation autres que les préparations de laboratoire - de préparation d'aminoacides chiraux nécessitent donc des synthèses énantiosélectives, c'est-à-dire, des synthèses ne conduisant qu'au seul énantiomère souhaité et ce avec une pureté énantiomérique élevée.

Des procédés de préparation d'aminoacides à partir d'hydantoïnes sont connus par exemple dans les brevets JP 60224661 et JP 62103049. Ces procédés conduisent cependant à des aminoacides racémiques.

En revanche, le brevet EP-A-739978 présente quant à lui un procédé de préparation d'aminoacides optiquement purs à partir d'hydantoïnes racémiques.

Ce type de procédé possède l'inconvénient de mettre en oeuvre une réaction enzymatique et de comporter plusieurs étapes. Ceci a une conséquence directe sur la complexité du procédé industriel, sur les rendements en produit obtenu et sur les coûts de fabrication.

Un objet de la présente invention est de proposer un procédé de préparation d'aminoacides chiraux à partir d'hydantoïnes racémiques ne comportant pas les inconvénients cités ci-dessus.

Un objet de la présente invention est de proposer un procédé de préparation d'aminoacides substantiellement énantiomériquement purs à partir d'hydantoïnes racémiques.

Un autre objet de la présente invention est de proposer un procédé de préparation d'aminoacides substantiellement énantiomériquement purs à partir d'hydantoïnes racémiques, le dit procédé ne comportant qu'une seule étape ("one-pot reaction"), sans isolement de l'hydantoïne chirale intermédiaire.

Un autre objet de la présente invention est de proposer un procédé de préparation d'aminoacides substantiellement énantiomériquement purs à partir d'hydantoïnes racémiques, avec un rendement élevé.

Un objet supplémentaire de la présente invention est de proposer un procédé de préparation d'aminoacides substantiellement énantiomériquement purs à partir d'hydantoïnes racémiques, procédé facilement industrialisable et de coût peu élevé.

Il a maintenant été découvert que tous ces buts peuvent être atteints en totalité ou en partie grâce au procédé selon l'invention, dont la description est présentée ci-dessous.

### Brève description de l'invention

La présente invention consiste en un nouveau procédé de préparation d'aminoacides chiraux de formule (I) :

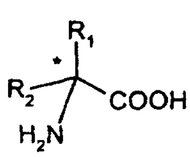

**(I)**

caractérisé en ce que l'on met en contact une hydantoïne racémique de formule (II) :

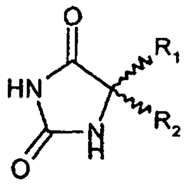

(II)

avec un agent de dédoublement énantiomérique,
composés de formules (I) et (II) dans lesquels :

- $R_1$ et $R_2$ sont différents et sont choisis parmi :
  - un radical alkyle ou haloalkyle contenant de 1 à 6 atomes de carbone en chaîne linéaire ou ramifiée ;
  - un radical alkoxyalkyle, alkylthioalkyle, alkylsulfonylalkyle, monoalkylaminoalkyle, alcényle ou alcynyle contenant de 2 à 6 atomes de carbone en chaîne linéaire ou ramifiée ;
  - un radical dialkylaminoalkyle ou cycloalkyle contenant de 3 à 7 atomes de carbone en chaîne linéaire ou ramifiée ;
  - un radical aryle, c'est-à-dire phényle, naphtyle, thiényle, furyle, pyridyle, benzothiényle, benzofuryle, quinolinyle, isoquinolinyle, ou méthylènedioxyphényle, éventuellement substitué par 1 à 3 groupements choisis parmi $R_6$ ; et
  - un radical arylalkyle, aryloxyalkyle, arylthioalkyle ou arylsulfonylalkyle, les termes aryle et alkyle ayant les définitions données ci-dessus ;

    ou bien
  - $R_1$ et $R_2$ peuvent former, avec le carbone auquel ils sont liés sur le cycle, un carbocycle ou un hétérocycle comprenant de 5 à 7 atomes, ces cycles pouvant être fusionnés avec un phényle, éventuellement substitué par 1 à 3 groupes choisis parmi $R_6$ ;

- $R_6$ représente un radical choisi parmi :
  - un atome d'halogène ;
  - un radical alkyle, haloalkyle, alkoxy, haloalkoxy, alkylthio, haloalkylthio ou alkylsulfonyle contenant de 1 à 6 atomes de carbone ;
  - un radical cycloalkyle, halocycloalkyle, alcényloxy, alcynyloxy, alcénylthio, alcynylthio contenant de 3 à 6 atomes de carbone ;
  - le groupe nitro ou cyano ;
  - un radical amino éventuellement mono ou disubstitué par un radical alkyle ou acyle contenant de 1 à 6 atomes de carbone ou alkoxycarbonyle contenant de 2 à 6 atomes de carbone ; et
  - un radical phényle, phénoxy ou pyridyloxy, ces radicaux étant éventuellement substitués par 1 à 3 groupements, identiques ou différents, choisis parmi $R_7$ ; et

- $R_7$ représente un radical choisi parmi :
  - un atome d'halogène choisi parmi fluor, chlore, brome et iode ;
  - un radical alkyle, linéaire ou ramifié, contenant de 1 à 6 atomes de carbone ;
  - un radical alkoxy ou alkylthio, linéaire ou ramifié, contenant de 1 à 6 atomes de carbone ;
  - un radical haloalkoxy ou haloalkylthio, linéaire ou ramifié, contenant de 1 à 6 atomes de carbone ;
  - un radical nitrile ; et
  - un radical nitro.

Les aminoacides chiraux ainsi obtenus peuvent servir comme intermédiaires de synthèse dans l'élaboration de matières actives chirales utiles notamment en thérapeutique ou en agriculture. À titre d'exemple, ces aminoacides chiraux peuvent être utilisés comme intermédiaires dans la préparation de certaines 2-imidazoline-5-ones et 2-imidazoline-5-thiones fongicides décrites dans le brevet EP-A-0 629 616.

## Description détaillée de l'invention

La présente invention concerne un nouveau procédé de préparation d'aminoacides chiraux de formule (I) :

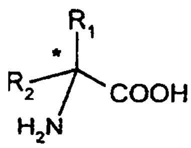

(I)

dans laquelle :

- $R_1$ et $R_2$ sont différents et sont choisis parmi :
  - un radical alkyle ou haloalkyle contenant de 1 à 6 atomes de carbone en chaîne linéaire ou ramifiée ;
  - un radical alkoxyalkyle, alkylthioalkyle, alkylsulfonylalkyle, monoalkylaminoalkyle, alcényle ou alcynyle contenant de 2 à 6 atomes de carbone en chaîne linéaire ou ramifiée ;
  - un radical dialkylaminoalkyle ou cycloalkyle contenant de 3 à 7 atomes de carbone en chaîne linéaire ou ramifiée ;
  - un radical aryle, c'est-à-dire phényle, naphtyle, thiényle, furyle, pyridyle, benzothiényle, benzofuryle, quinolinyle, isoquinolinyle, ou méthylènedioxyphényle, éventuellement substitué par 1 à 3 groupements choisis parmi $R_6$ ; et
  - un radical arylalkyle, aryloxyalkyle, arylthioalkyle ou arylsulfonylalkyle, les termes aryle et alkyle ayant les définitions données ci-dessus;
    ou bien
  - $R_1$ et $R_2$ peuvent former, avec le carbone auquel ils sont liés sur le cycle, un carbocycle ou un hétérocycle comprenant de 5 à 7 atomes, ces cycles pouvant être fusionnés avec un phényle, éventuellement substitué par 1 à 3 groupes choisis parmi $R_6$ ;

- $R_6$ représente un radical choisi parmi :
  - un atome d'halogène;
  - un radical alkyle, haloalkyle, alkoxy, haloalkoxy, alkylthio, haloalkylthio ou alkylsulfonyle contenant de 1 à 6 atomes de carbone ;
  - un radical cycloalkyle, halocycloalkyle, alcényloxy, alcynyloxy, alcénylthio, alcynylthio contenant de 3 à 6 atomes de carbone ;
  - le groupe nitro ou cyano ;
  - un radical amino éventuellement mono ou disubstitué par un radical alkyle ou acyle contenant de 1 à 6 atomes de carbone ou alkoxycarbonyle contenant de 2 à 6 atomes de carbone ; et
  - un radical phényle, phénoxy ou pyridyloxy, ces radicaux étant éventuellement substitués par 1 à 3 groupements, identiques ou différents, choisis parmi $R_7$ ; et

- $R_7$ représente un radical choisi parmi :
  - un atome d'halogène choisi parmi fluor, chlore, brome et iode ;
  - un radical alkyle, linéaire ou ramifié, contenant de 1 à 6 atomes de carbone ;
  - un radical alkoxy ou alkylthio, linéaire ou ramifié, contenant de 1 à 6 atomes de carbone ;
  - un radical haloalkoxy ou haloalkylthio, linéaire ou ramifié, contenant de 1 à 6 atomes de carbone ;
  - un radical nitrile ; et
  - un radical nitro.

Selon un aspect préféré de l'invention, les aminoacides chiraux de formule (I) sont tels que :

- $R_1$ représente un radical aryle, éventuellement substitué par 1 à 3 groupements $R_6$ tels que définis précédemment, et
- $R_2$ représente un radical alkyle ou haloalkyle contenant de 1 à 6 atomes de carbone en chaîne linéaire ou ramifiée.

Selon un aspect tout particulièrement préféré de l'invention, les aminoacides de formule (I) sont tels que :

- $R_1$ représente un radical phényle, éventuellement substitué par un groupement $R_6$ tel que défini précédemment, et
- $R_2$ représente un radical alkyle choisi parmi méthyle, éthyle, propyle linéaire ou ramifié, butyle linéaire ou ramifié, pentyle linéaire ou ramifié et hexyle linéaire ou ramifié.

L'astérisque (*) porté par le carbone asymétrique dans la formule (I) signifie que l'aminoacide est chiral, c'est-à-dire que l'aminoacide est de configuration *S* ou *R*.
Le procédé selon l'invention permet d'obtenir des aminoacides chiraux. Par "aminoacides chiraux", on entend les énantiomères substantiellement purs, soit de configuration *S*, soit de configuration *R*.
Le terme "substantiellement pur" signifie que l'excès énantiomérique de l'énantiomère considéré est supérieur à 80%, plus particulièrement supérieur à 90%. Certaines conditions opératoires du procédé selon l'invention conduisent également à un excès énantiomérique de 100% ; c'est-à-dire que, dans ce cas, l'énantiomère obtenu est pur, l'autre forme énantiomérique n'étant pas décelable.
Par "excès énantiomérique", on entend le ratio de l'excès de l'énantiomère désiré par rapport à l'énantiomère non désiré.
Ce ratio est calculé selon l'une des équations suivantes :

$$\%\ e.e.(S) = \frac{[S]-[R]}{[R]+[S]} \times 100 \qquad \%\ e.e.(R) = \frac{[R]-[S]}{[R]+[S]} \times 100$$

dans lesquelles :

- % e.e.(S) représente l'excès énantiomérique en isomère *S*,
- % e.e.(R) représente l'excès énantiomérique en isomère *R*,
- [*S*] représente la concentration en isomère S, et
- [*R*] représente la concentration en isomère *R*.

Le procédé selon l'invention est caractérisé en ce que l'on met en contact un agent de dédoublement énantiomérique et une hydantoïne racémique de formule (II) :

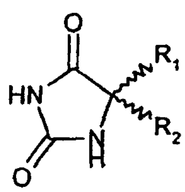
**(II)**

dans laquelle $R_1$ et $R_2$ sont tels que définis précédemment.
Par "hydantoïne racémique", on entend une hydantoïne de formule (II) substantiellement non optiquement active, c'est-à-dire dont un énantiomère n'est pas substantiellement prépondérant par rapport à l'autre.
Le procédé de l'invention consiste donc à :

- (a) solubiliser une hydantoïne racémique de formule (II) telle que définie précédemment, en milieu basique ;
- (b) ajouter un agent de dédoublement énantiomérique ;
- (c) séparer l'agent de dédoublement du milieu réactionnel ;
- (d) hydrolyser le milieu réactionnel en milieu basique afin de libérer le sel basique de l'aminoacide chiral souhaité ;

les opérations (a) et (b) pouvant être effectuées simultanément ou consécutivement.
Chacune des opérations (a) à (d) définies ci-dessus fait appel à des techniques connues de l'homme du métier, spécialisé en synthèse organique.
L'ensemble des opérations (a) à (d) est avantageusement réalisé en une seule étape ("one-pot-reaction"), c'est-à-dire sans isolement des intermédiaires obtenus après chacune des opérations, c'est-à-dire encore dans le même appareillage réactionnel.
La réalisation du procédé selon l'invention en une seule étape offre donc un avantage tout particulier lorsque le procédé est conduit à l'échelon industriel.

La première opération du procédé de l'invention consiste à mélanger, sous agitation et dans un solvant convenablement choisi, une hydantoïne racémique de formule (II) telle que définie précédemment, un agent de dédoublement et une base. Le milieu réactionnel peut être éventuellement chauffé pour permettre la solubilisation complète des réactifs.

Le précipité obtenu est séparé et mélangé à une solution aqueuse basique.

La solution ainsi obtenue est traitée afin de séparer l'agent de dédoublement. Cette séparation peut être effectuée par tout procédé connu de l'homme du métier ; de manière particulièrement avantageuse, cette séparation est effectuée par distillation, de préférence sous pression réduite. On procède de même pour séparer l'agent de dédoublement contenu dans le filtrat.

De cette façon, l'agent de dédoublement peut être séparé et récupéré de façon substantiellement quantitative et être directement réutilisé, sans autre traitement supplémentaire, dans un nouveau cycle de préparation d'aminoacide chiral.

Après élimination de l'agent de dédoublement, la solution de sel d'hydantoïne est soumise à hydrolyse. Cette opération peut être avantageusement réalisée par simple chauffage du milieu réactionnel.

Enfin, l'aminoacide chiral souhaité est récupéré selon des méthodes classiques, après neutralisation du milieu.

L'agent de dédoublement utilisé dans le procédé de la présente invention peut être tout agent de dédoublement connu de l'homme du métier spécialisé dans l'art de la synthèse asymétrique. Cet agent de dédoublement est de tout type convenable pour la réaction envisagée et est un composé chiral (ou asymétrique) de configuration précise et connue définie généralement par les termes dextrogyre ou lévogyre qui reflètent l'activité optique de ce composé. Cet agent de dédoublement peut être choisi, par exemple, parmi les agents de dédoublement chiraux, comme par exemple les amines chirales, telles que la quinine, la cinchonidine, la déhydroabiéthylamine, l'éphédrine, le 2-amino-1-phényl-1,3-propanediol, 1'$\alpha$-méthylbenzylamine, l'$\alpha$-(1-naphtyl)-éthylamine, ou le 2-phénylglycinol, et les acides chiraux, tels que l'acide tartrique, l'acide dibenzoyltartrique, l'acide malique, l'acide camphosulfonique, l'acide mandélique, ou le phencyphos. Selon un aspect tout particulièrement préféré de l'invention, l'agent de dédoublement chiral utilisé pour le procédé de l'invention est l'$\alpha$-méthylbenzylamine dextrogyre (+)-$\alpha$-MBA ou l'$\alpha$-méthylbenzylamine lévogyre (-)-$\alpha$-MBA selon que l'on souhaite préparer un aminoacide dextrogyre, respectivement lévogyre. L'utilisation de cet agent de dédoublement est par exemple décrit dans le brevet WO-A-92/08702 ou dans la publication de G. Coquerel et coll., *Chirality, 4*, (1992), 400-403.

De façon surprenante, le procédé de préparation d'aminoacides chiraux selon l'invention peut être réalisé en utilisant au plus un équivalent, par exemple de 0,2 à 1 équivalent, d'agent de dédoublement par rapport à la quantité d'hydantoïne racémique, et ce, en introduisant une base dans le milieu réactionnel lors de la mise en contact de l'hydantoïne racémique avec l'agent de dédoublement.

Cette base peut être une base organique ou une base inorganique. Parmi les bases inorganiques préférées pour le procédé selon l'invention, on peut citer les hydroxydes, par exemple les hydroxydes des métaux alcalins ou alcalino-terreux, par exemple l'hydroxyde de sodium ou bien encore l'hydroxyde de potassium. Comme base organique pouvant être utilisée pour le procédé de l'invention, on peut mentionner les amines, de préférence les amines tertiaires, par exemple la triéthylamine.

La base employée pour le procédé de l'invention est présente dans le milieu réactionnel en une quantité comprise entre 0,2 et 0,8 équivalent par rapport à la quantité d'hydantoïne racémique présente au départ.

Comme base préférée pour le procédé décrit dans la présente demande, on peut citer les hydroxydes de métaux alcalins ou alcalino-terreux, de préférence l'hydroxyde de sodium ou l'hydroxyde de potassium. De manière tout particulièrement préférée la base utilisée est l'hydroxyde de sodium. La quantité de base utilisée varie entre 0,2 et 0,8 équivalents de base par rapport à la quantité d'hydantoïne racémique de départ, de préférence entre 0,4 et 0,6, par exemple 0,5 équivalents.

Selon un aspect du procédé de la présente invention, l'hydantoïne racémique de formule (II) est mise en solution. Cette dissolution est réalisée dans un solvant organique ou inorganique, ou dans un mélange de solvants organiques ou inorganiques ou encore dans un mélange de solvants organiques et inorganiques. Par solvant organique, on entend préférentiellement les solvants polaires protiques ou aprotiques, tels que les alcools ou les cétones, par exemple le méthanol, l'éthanol ou la diméthylcétone. Par solvant inorganique, on entend là encore préférentiellement les solvants polaires, par exemple l'eau.

Pour la réalisation du procédé selon l'invention, on choisira préférentiellement l'eau comme solvant de l'hydantoïne, de préférence encore un système eau/co-solvant, le dit co-solvant étant avantageusement choisi parmi le méthanol, l'éthanol ou l'acétone.

Les proportions des différents constituants de ce solvant, eau/co-solvant, sont choisies de telle sorte qu'elles permettent d'obtenir une concentration d'hydantoïne dans le milieu réactionnel comprise entre 5 et 30% en poids.

Pour cela, on choisit un rapport des quantités eau/co-solvant compris entre 90/10 et 30/70, selon la nature du co-solvant.

On peut, par exemple, utiliser un solvant constitué d'un mélange d'eau et d'éthanol dans un rapport 70/30.

La nature du solvant de l'hydantoïne racémique est particulièrement intéressante : en effet, un pouvoir de solvatation élevé conduit à une concentration en hydantoïne élevée dans le milieu réactionnel, et par voie de conséquence limite considérablement le volume d'effluents. Ce dernier aspect est tout particulièrement important dans le cas d'un procédé industriel.

La dissolution de l'hydantoïne peut également être facilitée par chauffage du milieu réactionnel. À titre d'exemple, le milieu réactionnel peut être chauffé à des températures comprises entre 40°C et 80°C, par exemple entre 50°C et 60°C.

Après dissolution de l'hydantoïne racémique, ajout dans le milieu réactionnel de l'agent de dédoublement, et éventuellement refroidissement de l'ensemble, à une température telle qu'elle permette la précipitation des composés les moins solubles, le précipité obtenu est séparé.

À une solution aqueuse de ce précipité, on ajoute un excès d'une base en solution aqueuse également. La base utilisée est une base inorganique, par exemple de l'hydroxyde de sodium ou de potassium ou encore de l'hydroxyde d'ammonium. L'excès de base ajoutée est compris entre 1 et 10 équivalents par rapport à la quantité d'hydantoïne racémique de départ.

Enfin, on sépare directement depuis le milieu réactionnel basique l'agent de dédoublement énantiomérique qui peut ensuite être réutilisé pour le procédé de l'invention. Cette séparation est réalisée par tout procédé connu de l'homme du métier. Selon un aspect tout particulièrement préféré de l'invention, cette séparation est réalisée par distillation.

Le milieu réactionnel contient alors une solution de sel basique d'hydantoïne chirale, libre de tout agent de dédoublement, sel qui n'est pas isolé, mais transformé directement en sel de l'aminoacide chiral correspondant. Cette hydrolyse peut ici encore être réalisée selon tout procédé connu dans la littérature. Par exemple l'hydrolyse est effectuée par simple chauffage, à des températures variant de 50°C à 250°C, de préférence de 100°C à 200°C, avec des durées de réaction variant de quelques minutes à plus d 20 heures, selon la température choisie.

Enfin, l'aminoacide chiral attendu est isolé du milieu réactionnel selon les techniques classiques employées dans ce domaine, telles que neutralisation, lavage(s), recristallisation(s), distillation(s), séchage(s), etc. Une ou plusieurs de ces techniques pourront être effectuées simultanément ou consécutivement, dans des conditions opératoires connues de l'homme du métier qui saura choisir les réactifs et les conditions réactionnelles adéquates et adaptées à chaque cas.

- Les aminoacides chiraux de formule (I) définie précédemment obtenus selon le précédé de l'invention trouvent une application tout particulièrement intéressante en tant qu'intermédiaires de synthèse dans l'élaboration de matières actives chirales utiles notamment en thérapeutique ou en agriculture.

Par exemple, les aminoacides chiraux de formule (I) peuvent être utilisés comme intermédiaires dans la préparation de certaines 2-imidazoline-5-ones et 2-imidazoline-5-thiones fongicides décrites dans le brevet EP-A-0 629 616 de formule (A) :

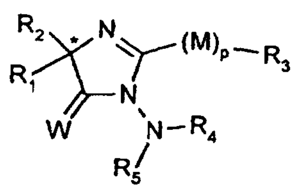

(A)

dans laquelle $R_1$ et $R_2$ sont tels que définis précédemment pour les aminoacides de formule (I) et

- W représente un atome d'oxygène ou de soufre, ou un groupe S=O ;
- M représente un atome d'oxygène ou de soufre, ou un radical $CH_2$, éventuellement halogéné ;
- p est un nombre entier égal à 0 ou 1 ;
- $R_3$ représente :
  - un hydrogène ou un radical $C_1$-$C_2$ alkyle éventuellement halogéné, lorsque p égale 0 ou $(M)_p$ est un radical $CH_2$,
  - un radical $C_1$-$C_2$ alkyle éventuellement halogéné, lorsque $(M)_p$ représente un atome d'oxygène ou de soufre ;

- $R_4$ représente :
    - l'atome d'hydrogène, ou
    - un radical alkyle contenant de 1 à 6 atomes de carbone, ou
    - un radical alkoxyalkyle, alkylthioalkyle, haloalkyle, cyanoalkyle, thiocyanatoalkyle, alcényle ou alcynyle contenant de 2 à 6 atomes de carbone, ou
    - un radical dialkylaminoalkyle, alkoxycarbonylalkyle, ou N-alkylcarbamoylalkyle contenant de 3 à 6 atomes de carbone, ou
    - un radical N,N-dialkylcarbamoylalkyle contenant de 4 à 8 atomes de carbone, ou
    - un radical aryle, comprenant phényle, naphtyle, thiényle, furyle, pyridyle, pyrimidyle, pyridazinyle, pyrazinyle, benzothiényle, benzofuryle, quinolinyle, isoquinolinyle, ou méthylène dioxyphényle, éventuellement substitué par 1 à 3 groupements choisis parmi $R_6$, ou
    - un radical arylalkyle, aryloxyalkyle, arylthioalkyle ou arylsulfonylalkyle, les termes aryle et alkyle ayant les définitions données ci-dessus ;

- $R_5$ représente :
    - l'hydrogène, ou un radical alkyle, haloalkyle, alkylsulfonyle, haloalkylsulfonyle contenant de 1 à 6 atomes de carbone ou,
    - un radical alkoxyalkyle, alkylthioakyle, acyle, alcényle, alcynyle, haloacyle, alkoxycarbonyle, haloalkoxycarbonyle, alkoxyalkylsulfonyle, cyanoalkylsulfonyle contenant de 2 à 6 atomes de carbone ou
    - un radical alkoxyalkoxycarbonyle, alkylthioalkoxycarbonyle, cyanoalkoxycarbonyle contenant de 3 à 6 atomes de carbone ou,
    - le radical formyle ou un radical cycloalkyle, alkoxyacyle, alkylthioacyle, cyanoacyle, alcénylcarbonyle, alcynylcarbonyle contenant de 3 à 6 atomes de carbone ou,
    - un radical cycloalkylcarbonyle contenant de 4 à 8 atomes de carbone ou,
    - un radical phényle; arylalkylcarbonyle, notamment phénylacétyle et phénylpropionyle, arylcarbonyle, notamment benzoyle, éventuellement substitué par 1 à 3 groupes parmi $R_6$, thiénylcarbonyle, furylcarbonyle, pyridylcarbonyle, benzyloxycarbonyle, furfuryloxycarbonyle, tetrahydrofurfuryloxycarbonyle, thiénylméthoxycarbonyle, pyridylméthoxycarbonyle, phénoxycarbonyle ou phénylthiolcarbonyle, le radical phényle étant lui-même éventuellement substitué par 1 à 3 groupement choisis parmi $R_6$, alkylthiolcarbonyle, haloalkylthiolcarbonyle, alkoxyalkylthiolcarbonyle, cyanoalkylthiolcarbonyle, benzylthiolcarbonyle, furfurylthiolcarbonyle, tétrahydrofurfurylthiolcarbonyle, thiénylméthylthiolcarbonyle, pyridylméthylthiolcarbonyle, ou arylsulfonyle ou
    - un radical carbamoyle éventuellement mono ou disubstitué par :
        - un groupe alkyle ou haloalkyle contenant de 1 à 6 atomes de carbone,
        - un groupe cycloalkyle, alcényle ou alcynyle contenant de 3 à 6 atomes de carbone,
        - un groupe alkoxyalkyle, alkylthioalkyle ou cyanoalkyle contenant de 2 à 6 atomes de carbone ou,
        - un phényle éventuellement substitué par 1 à 3 groupement $R_6$ ;
    - un groupement sulfamoyle éventuellement mono ou disubstitué par :
        - un groupe alkyle ou haloalkyle contenant de 1 à 6 atomes de carbone,
        - un groupe cycloalkyle, alcényle ou alcynyle contenant de 3 à 6 atomes de carbone,
        - un groupe alkoxyalkyle, alkylthioalkyle ou cyanoalkyle de 2 à 6 atomes de carbone ou
        - un phényle éventuellement substitué par 1 à 3 groupement $R_6$ ;
    - un groupe alkylthioalkylsulfonyle contenant de 3 à 8 atomes de carbone ou cycloalkylsulfonyle contenant de 3 à 7 atomes de carbone ;
    - $R_4$ et $R_5$ pris ensemble peuvent également former avec l'atome d'azote auquel ils sont attachés un groupe pyrrolidino, pipéridino, morpholino ou pipérazino éventuellement substitué par un radical alkyle contenant de 1 à 3 atomes de carbone.

- $R^6$ représente :
    - un atome d'halogène ou
    - un radical alkyle, haloalkyle, alkoxy, haloalkoxy, alkylthio, haloalkylthio ou alkylsulfonyle contenant de 1 à 6 atomes de carbone ou

- un radical cycloalkyle, halocycloalkyle, alcényloxy, alcynyloxy, alcénylthio, alcynylthio contenant de 3 à 6 atomes de carbone ou
- le groupe nitro ou cyano ou
- un radical amino éventuellement mono ou disubstitué par un radical alkyle ou acyle contenant de 1 à 6 atomes de carbone ou alkoxycarbonyle contenant de 2 à 6 atomes de carbone,
- un radical phényle, phénoxy ou pyridyloxy, ces radicaux étant éventuellement substitués par 1 à 3 groupements, identiques ou différents, choisis parmi $R_7$,

• $R_7$ représente :

- un atome d'halogène choisi parmi le fluor, le chlore, le brome, l'iode ou,
- un radical alkyle contenant de 1 à 6 atomes de carbone, ou
- un radical alkoxy ou alkylthio contenant de 1 à 6 atomes de carbone ou,
- un radical haloalkoxy ou haloalkylthio contenant de 1 à 6 atomes de carbone ou,
- un radical nitrile ou nitro ;

Le procédé de préparation des composés de formule (A) peut être représenté par le schéma suivant :

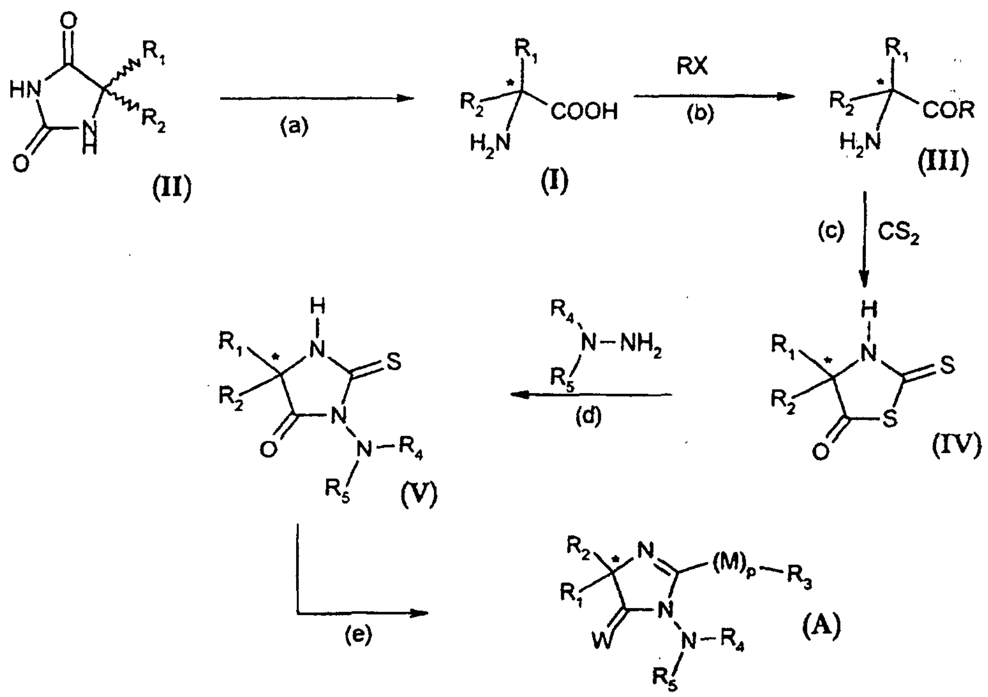


schéma dans lequel, les radicaux $R_1$, $R_2$, $R_3$, $R_4$, $R_5$, M, p et W sont tels que définis précédemment, R représente un radical hydroxy, alkoxy contenant de 1 à 6 atomes de carbone, benzyloxy, un radical amino, alkylamino ou dialkylamino, un radical alkylamino contenant de 1 à 6 atomes de carbone, et X représente un groupement partant tel qu'un atome d'halogène, choisi parmi chlore, brome et iode, ou un radical sulfate, ou alkylsulfonyloxy ou arylsulfonyloxy.

Dans le schéma précédent

- l'étape (a) est le procédé de la présente invention et est exemplifié dans la suite de la présente description;
- les étapes (b), (c) et (d) sont décrites dans le brevet WO-98/03490 dont le détail est incorporé ici par référence ;
- l'étape (e) est décrite dans le brevet EP-A-0 629 616 dont le détail est incorporé ici par référence.

Le procédé global de synthèse des composés de formule (A), à partir d'hydantoïnes racémiques de formule (II) via les intermédiaires de formule (I), est nouveau et à ce titre est compris dans le champ de la présente invention.

Les exemples qui suivent permettront d'illustrer et de mieux apprécier les objets et avantages du procédé de l'invention sans toutefois limiter l'étendue de celle-ci.

**Exemple 1 :**

Dans un solvant constitué d'eau et d'éthanol dans un rapport 70/30 et sous agitation, on mélange 15,7 mmoles de 5-méthyl-5-phénylhydantoïne racémique, 15,7 mmoles de R-(+)-α-méthylbenzylamine et 7,85 mmoles d'hydroxyde de sodium.
On chauffe le milieu réactionnel à 50°C pendant 1h30 puis on le refroidit jusqu'à 10°C.
Le précipité est filtré puis lavé à l'eau.
On dissout ensuite ce précipité dans de l'eau en présence de soude (0,3 équivalent en poids de précipité). La solution obtenue est engagée dans une distillation azéotropique sous pression réduite (400 mbar), afin de récupérer quantitativement la R-(+)-α-méthylbenzylamine.
Le milieu réactionnel est alors porté à 140°C pendant 4h.
Après refroidissement jusqu'à 25°C, on acidifie le milieu réactionnel avec une solution aqueuse d'acide chlorhydrique à 33%.
On filtre alors le précipité d'aminoacide chiral obtenu qui est ensuite lavé (eau, acétone) puis séché sous vide.
On obtient l'aminoacide chiral dextrogyre attendu avec un rendement de 32% par rapport à l'hydantoïne racémique de départ et un excès énantiomérique de 97%.

**Exemple 2 :**

Dans un solvant constitué d'eau et de méthanol dans un rapport 70/30 et sous agitation, on mélange de la 5-méthyl-5-phénylhydantoïne racémique, de la R-(+)-α-méthylbenzylatnine (0,9 équivalent par rapport à la quantité de 5-méthyl-5-phénylhydantoïne racémique) et de la soude (0,5 équivalent par rapport à la quantité de 5-méthyl-5-phénylhydantoïne racémique).
On chauffe le milieu réactionnel à 55°C pendant 30 min. puis on le refroidit jusqu'à 20°C.
On filtre puis on lave avec de l'eau le précipité obtenu.
On dissout ensuite ce précipité dans de l'eau en présence de soude (0,3 équivalent en poids de précipité), à partir de la solution obtenue, on sépare la R-(+)-α-méthylbenzylamine par distillation.
On chauffe ensuite le milieu réactionnel à 160°C pendant 4h.
Après refroidissement jusqu'à 25°C, on acidifie le milieu réactionnel avec une solution aqueuse d'acide chlorhydrique à 33%.
On filtre alors le précipité d'aminoacide chiral obtenu puis on le lave et enfin on le sèche.
On obtient la S-(+)-méthylphénylglycine avec un rendement de 33% par rapport à l'hydantoïne racémique de départ et un excès énantiomérique de 98%.

**Revendications**

1. Procédé de préparation d'aminoacides chiraux de formule (I) :

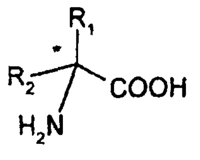

(I)

dans laquelle :

- $R_1$ et $R_2$ sont différents et sont choisis parmi :
  - un radical alkyle ou haloalkyle contenant de 1 à 6 atomes de carbone en chaîne linéaire ou ramifiée ;
  - un radical alkoxyalkyle, alkylthioalkyle, alkylsulfonylalkyle, monoalkylaminoalkyle, alcényle ou alcynyle contenant de 2 à 6 atomes de carbone en chaîne linéaire ou ramifiée ;
  - un radical dialkylaminoalkyle ou cycloalkyle contenant de 3 à 7 atomes de carbone en chaîne linéaire ou ramifiée ;
  - un radical aryle, c'est-à-dire phényle, naphtyle, thiényle, furyle, pyridyle, benzothiényle, benzofuryle, quinolinyle, isoquinolinyle, ou méthylènedioxyphényle, éventuellement substitué par 1 à 3 groupements choisis parmi $R_6$; et

- un radical arylalkyle, aryloxyalkyle, arylthioalkyle ou arylsulfonylalkyle, les termes aryle et alkyle ayant les définitions données ci-dessus ;

  ou bien

- $R_1$ et $R_2$ peuvent former, avec le carbone auquel ils sont liés sur le cycle, un carbocycle ou un hétérocycle comprenant de 5 à 7 atomes, ces cycles pouvant être fusionnés avec un phényle, éventuellement substitué par 1 à 3 groupes choisis parmi $R_6$;

• $R_6$ représente un radical choisi parmi :

- un atome d'halogène ;
- un radical alkyle, haloalkyle, alkoxy, haloalkoxy, alkylthio, haloalkylthio ou alkylsulfonyle contenant de 1 à 6 atomes de carbone ;
- un radical cycloalkyle, halocycloalkyle, alcényloxy, alcynyloxy, alcénylthio, alcynylthio contenant de 3 à 6 atomes de carbone ;
- le groupe nitro ou cyano ;
- un radical amino éventuellement mono ou disubstitué par un radical alkyle ou acyle contenant de 1 à 6 atomes de carbone ou alkoxycarbonyle contenant de 2 à 6 atomes de carbone ; et
- un radical phényle, phénoxy ou pyridyloxy, ces radicaux étant éventuellement substitués par 1 à 3 groupements, identiques ou différents, choisis parmi $R_7$ ; et

• $R_7$ représente un radical choisi parmi :

- un atome d'halogène choisi parmi fluor, chlore, brome et iode;
- un radical alkyle, linéaire ou ramifié, contenant de 1 à 6 atomes de carbone;
- un radical alkoxy ou alkylthio, linéaire ou ramifié, contenant de 1 à 6 atomes de carbone;
- un radical haloalkoxy ou haloalkylthio, linéaire ou ramifié, contenant de 1 à 6 atomes de carbone ;
- un radical nitrile ; et
- un radical nitro,

**caractérisé en ce que** l'on met en contact une hydantoïne racémique de formule (II):

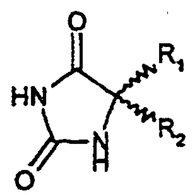

(II)

dans laquelle R1 et R2 sont tels que définis pour la formule (I),
avec un agent de dédoublement énantiomérique et une base.

**2.** Procédé selon la revendication 1, **caractérisé en ce qu'**il consiste à :

- (a) solubiliser une hydantoïne racémique de formule (II) selon la revendication 1, en milieu basique ;
- (b) ajouter un agent de dédoublement énantiomérique ;
- (c) séparer l'agent de dédoublement du milieu réactionnel ;
- (d) hydrolyser le milieu réactionnel en milieu basique afin de libérer le sel basique de l'aminoacide chiral souhaité ;

les opérations (a) et (b) pouvant être effectuées simultanément ou consécutivement.

**3.** Procédé selon l'une des revendications 1 ou 2, **caractérisé en ce qu'**il est réalisé en utilisant de 0,2 à 1 équivalent d'agent de dédoublement par rapport à la quantité d'hydantoïne racémique employée.

**4.** Procédé selon l'une des revendications 1 à 3, **caractérise en ce qu'**il est réalisé en une seule étape ("one pot reaction").

**5.** Procédé selon l'une des revendications 1 à 4, **caractérisé en ce que** l'agent de dédoublement est une amine chirale ou un acide chiral.

**6.** Procédé selon la revendication 5, **caractérisé en ce que** l'agent de dédoublement est la *R*-(+)-α-méthylbezylamine ou la *S*-(-)-α-méthylbenzylamine.

**7.** Procédé de préparation selon l'une des revendications précédentes, **caractérisé en ce qu'**il est réalisé en utilisant de 0,2 à 0,8 équivalents d'une base organique ou inorganique par rapport à la quantité d'hydantoine racémique employée.

**8.** Procédé selon l'une des revendications 1 à 7, **caractérisé en ce que** l'hydantoïne de formule (II) est mise en solution dans un système eau/co-solvant.

**9.** Procédé selon la revendication 8, **caractérisé en ce que** le co-solvant est choisi parmi le méthanol, l'éthanol et l'acétone.

**10.** Précédé selon l'une des revendications 8 et 9, **caractérisé en ce que** le rapport eau/co-solvant est tel qu'il permet d'obtenir une concentration du milieu réactionnel en hydantoine racémique comprise entre 5 et 30% en poids.

**11.** Procédé selon l'une des revendications 8 à 10, **caractérisé en ce que** le rapport eau/co-solvant est compris entre 90/10 et 30/70.

**12.** Procédé selon l'une des revendications précédentes, **caractérisé en ce que** l'agent de dédoublement est séparé après réaction, et récupéré de façon substantiellement quantitative pour être directement réutilisé; sans autre traitement supplémentaire, dans un nouveau cycle de préparation d'aminoacide chiral.

**13.** Procédé selon l'une quelconque des revendications précédentes, **caractérisé en ce que** les aminoacides chiraux de formule (I) sont tels que :

- R, représente un radical aryle, éventuellement substitué par 1 à 3 groupements $R_6$ tels que définis dans la revendication 1, et
- $R_2$ représente un radical alkyle ou haloalkyle contenant de 1 à 6 atomes de carbone en chaîne linéaire ou ramifiée.

**14.** Procédé selon la revendication 13, **caractérisé en ce que** les aminoacides chiraux de formules (I) sont tels que :

$R_1$ représente un radical phényle, éventuellement substitué par un groupement $R_6$ tel que défini précédemment, et
- $R_2$ représente un radical alkyle choisi parmi méthyle, éthyle, propyle linéaire ou ramifié, butyle linéaire ou ramifié, pentyle linéaire ou ramifié et hexyle linéaire ou ramifié.

**15.** Procédé de préparation des composés de formule (A) selon le schéma réactionnel suivant :

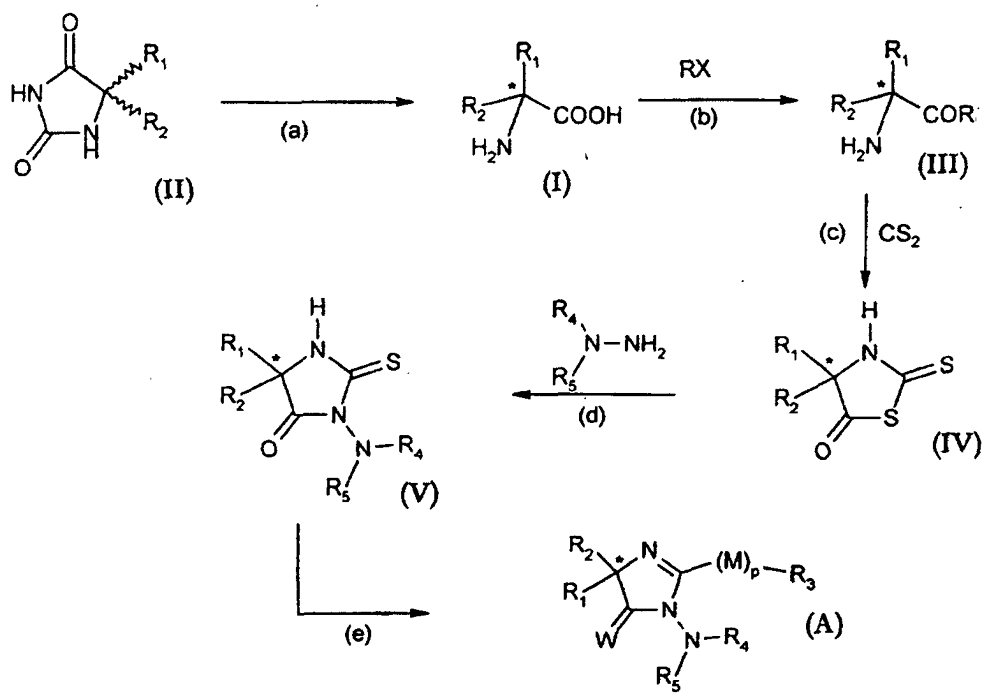


schéma dans lequel :

les radicaux $R_1$ et $R_2$ sont tels que définis dans l'une des revendications précédentes et :

- W représente un atome d'oxygène ou de soufre, ou un groupe S=O ;
- M représente un atome d'oxygène ou de soufre, ou un radical $CH_2$, éventuellement halogéné ;
- P est un nombre entier égal à 0 ou 1 ;
- $R_3$ représente :
  - un hydrogène ou un radical $C_1$-$C_2$ alkyle éventuellement halogéné, lorsque p égale 0 ou $(M)_p$ est un radical $CH_2$,
  - un radical $C_1$-$C_2$ alkyle éventuellement halogéné, lorsque $(M)_p$ représente un atome d'oxygène ou de soufre ;
- $R_4$ représente :
  - l'atome d'hydrogène, ou
  - un radical alkyle contenant de 1 à 6 atomes de carbone, ou
  - un alkoxyalkyle, alkylthioalkyle, haloalkyle, cyanoalkyle, thiocyanatoalkyle, alcényle ou alcynyle contenant de 2 à 6 atomes de carbone, ou
  - un radical dialkylaminoalkyle, alkoxycarbonylalkyle, ou N-alkylcarbamoylalkyle contenant de 3 à 6 atomes de carbone, ou
  - un radical N,N-dialkylcarbamoylalkyle contenant de 4 à 8 atomes de carbone, ou
  - un radical aryle, comprenant phényle, naphtyle, thiényle, furyle, pyridyle, pyrimidyle, pyridazinyle, pyrazinyle, benzothiényle, benzofuryle, quinolinyle, isoquinolinyle, ou méthylène dioxyphényle, éventuellement substitué par 1 à 3 groupements choisis parmi $R_6$, ou
  - un radical arylalkyle, aryloxyalkyle, arylthioalkyle ou arylsulfonylalkyle, les termes aryle et alkyle ayant les définitions données ci-dessus ;
- $R_5$ représente :
  - l'hydrogène, ou un radical alkyle, haloalkyle, alkylsulfonyle, haloalkylsulfonyle contenant de 1 à 6 atomes de carbone ou,
  - un radical alkoxyalkyle, alkylthioakyle, acyle, alcényle, alcynyle, haloacyle, alkoxycarbonyle, haloalkoxycarbonyle, alkoxyalkylsulfonyle, cyanoalkylsulfonyle contenant de 2 à 6 atomes de carbone ou

- un radical alkoxyalkoxycarbonyle, alkylthioalkoxycarbonyle, cyanoalkoxycarbonyle contenant de 3 à 6 atomes de carbone ou,
- le radical formyle ou un radical cycloalkyle, alkoxyacyle, alkylthioacyle, cyanoacyle, alcénylcarbonyle, alcynylcarbonyle contenant de 3 à 6 atomes de carbone ou,
- un radical cycloalkylcarbonyle contenant de 4 à 8 atomes de carbone ou,
- un radical phényle; arylalkylcarbonyle, notamment phénylacétyle et phénylpropionyle, arylcarbonyle, notamment benzoyle, éventuellement substitué par 1 à 3 groupes parmi $R_6$, thiénylcarbonyle, furylcarbonyle, pyridylcarbonyle, benzyloxycarbonyle, furfuryloxycarbonyle, tetrahydrofurfuryloxycarbonyle, thiénylméthoxycarbonyle, pyridylméthoxycarbonyle, phénoxycarbonyle ou phénylthiolcarbonyle, le radical phényle étant lui-même éventuellement substitué par 1 à 3 groupement choisis parmi $R_6$, alkylthiolcarbonyle, haloalkylthiolcarbonyle, alkoxyalkylthiolcarbonyle, cyanoalkylthiolcarbonyle, benzylthiolcarbonyle, furfurylthiolcarbonyle, tétrahydrofurfurylthiolcarbonyle, thiénylméthylthiolcarbonyle, pyridylméthylthiolcarbonyle, ou arylsulfonyle ou
- un radical carbamoyle éventuellement mono ou disubstitué par :
    - un groupe alkyle ou haloalkyle contenant de 1 à 6 atomes de carbone,
    - un groupe cycloalkyle, alcényle ou alcynyle contenant de 3 à 6 atomes de carbone,
    - un groupe alkoxyalkyle, alkylthioalkyle ou cyanoalkyle contenant de 2 à 6 atomes de carbone ou,
    - un phényle éventuellement substitué par 1 à 3 groupement $R_6$ ;
- un groupement sulfamoyle éventuellement mono ou disubstitué par :
    - un groupe alkyle ou haloalkyle contenant de 1 à 6 atomes de carbone,
    - un groupe cycloalkyle, alcényle ou alcynyle contenant de 3 à 6 atomes de carbone,
    - un groupe alkoxyalkyle, alkylthioalkyle ou cyanoalkyle de 2 à 6 atomes de carbone ou
    - un phényle éventuellement substitué par 1 à 3 groupement $R_6$ ;
- un groupe alkylthioalkylsulfonyle contenant de 3 à 8 atomes de carbone ou cycloalkylsulfonyle contenant de 3 à 7 atomes de carbone ;
- $R_4$ et $R_5$ pris ensemble peuvent également former avec l'atome d'azote auquel ils sont attachés un groupe pyrrolidino, pipéridino, morpholino ou pipérazino éventuellement substitué par un radical alkyle contenant de 1 à 3 atomes de carbone.

• $R^6$ représente :

- un atome d'halogène ou
- un radical alkyle, haloalkyle, alkoxy, haloalkoxy, alkylthio, haloalkylthio ou alkylsulfonyle contenant de 1 à 6 atomes de carbone ou
- un radical cycloalkyle, halocycloalkyle, alcényloxy, alcynyloxy, alcénylthio, alcynylthio contenant de 3 à 6 atomes de carbone ou
- le groupe nitro ou cyano ou
- un radical amino éventuellement mono ou disubstitué par un radical alkyle ou acyle contenant de 1 à 6 atomes de carbone ou alkoxycarbonyle contenant de 2 à 6 atomes de carbone,
- un radical phényle, phénoxy ou pyridyloxy, ces radicaux étant éventuellement substitués par 1 à 3 groupements, identiques ou différents, choisis parmi $R_7$,

• $R_7$ représente :

- un atome d'halogène choisi parmi le fluor, le chlore, le brome, l'iode ou,
- un radical alkyle contenant de 1 à 6 atomes de carbone, ou
- un radical alkoxy ou alkylthio contenant de 1 à 6 atomes de carbone ou,
- un radical haloalkoxy ou haloalkylthio contenant de 1 à 6 atomes de carbone ou,
- un radical nitrile ou nitro ;

• R représente un radical hydroxy, alkoxy contenant de 1 à 6 atomes de carbone, benzyloxy, un radical amino, alkylamino ou dialkylamino, un radical alkylamino contenant de 1 à 6 atomes de carbone, et
• X représente un groupement partant tel qu'un atome d'halogène, choisi parmi chlore, brome et iode, ou

un radical sulfate, ou alkylsulfonyloxy ou arylsulfonyloxy,

schéma **caractérisé en ce que** l'étape (a) est le procédé de transformation d'hydantoïnes racémiques de formule (II) en aminoacides de formule (I) selon l'une des revendications précédentes.

**Patentansprüche**

**1.** Verfahren zur Herstellung von chiralen Aminosäuren der Formel (I)

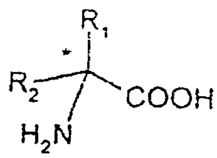 (I)

in der

- $R_1$ und $R_2$ verschieden sind und aus der folgenden Gruppe stammen:
    - Alkyl- oder Halogenalkylrest mit 1 bis 6 Kohlenstoffatomen in einer geraden oder verzweigten Kette,
    - Alkoxyalkyl-, Alkylthioalkyl-, Alkylsulfonylalkyl-, Monoalkylaminoalkyl-, Alkenyl- oder Alkinylrest mit 2 bis 6 Kohlenstoffatomen in einer geraden oder verzweigten Kette,
    - Dialkylaminoalkyl- oder Cycloalkylrest mit 3 bis 7 Kohlenstoffatomen in einer geraden oder verzweigten Kette,
    - gegebenenfalls durch 1 bis 3 Gruppen aus der Reihe $R_6$ substituierter Arylrest, also Phenyl, Naphthyl, Thienyl, Furyl, Pyridyl, Benzothienyl, Benzofuryl, Chinolinyl, Isochinolinyl oder Methylendioxyphenyl, und
    - Arylalkyl-, Aryloxyalkyl-, Arylthioalkyl- oder Arylsulfonylalkylrest, wobei die Ausdrücke Aryl und Alkyl wie oben definiert sind,<br>oder
    - $R_1$ und $R_2$ gemeinsam mit dem Kohlenstoff, an den sie in dem Cyclus gebunden sind, einen Carbocyclus oder Heterocyclus mit 5 bis 7 Atomen bilden können, wobei diese Cyclen mit einem gegebenenfalls durch 1 bis 3 Gruppen aus der Reihe $R_6$ substituiertem Phenyl anelliert sein können;
- $R_6$ einen Rest aus der folgenden Gruppe bedeutet:
    - Halogenatom,
    - Alkyl-, Halogenalkyl-, Alkoxy-, Halogenalkoxy-, Alkylthio-, Halogenalkylthio- oder Alkylsulfonylrest mit 1 bis 6 Kohlenstoffatomen,
    - Cycloalkyl-, Halogencycloalkyl-, Alkenyloxy-, Alkinyloxy-, Alkenylthio- oder Alkinylthiorest mit 3 bis 6 Kohlenstoffatomen,
    - Nitro- oder Cyanogruppe,
    - gegebenenfalls durch einen Alkyl- oder Acylrest mit. 1 bis 6 Kohlenstoffatomen oder Alkoxycarbonylrest mit 2 bis 6 Kohlenstoffatomen einfach oder zweifach substituierter Aminorest, und
    - Phenyl-, Phenoxy- oder Pyridyloxyrest, wobei diese Reste gegebenenfalls durch 1 bis 3 gleiche oder verschiedene Reste aus der Gruppe $R_7$ substituiert sind; und

- $R_7$ einen Rest aus der folgenden Gruppe bedeutet:
  - Halogenatom aus der Gruppe Fluor, Chlor, Brom und Iod,
  - geradkettiger oder verzweigter Alkylrest mit 1 bis 6 Kohlenstoffatomen,
  - geradkettiger oder verzweigter Alkoxy- oder Alkylthiorest mit 1 bis 6 Kohlenstoffatomen,
  - geradkettiger oder verzweigter Halogenalkoxyoder Halogenalkylthiorest mit 1 bis 6 Kohlenstoffatomen,
  - Nitrilrest, und
  - Nitrorest,

**dadurch gekennzeichnet, daß** man ein racemisches Hydantoin der Formel (II)

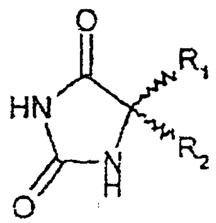

(II)

in der $R_1$ und $R_2$ wie für Formel (I) definiert sind, mit einem Enantiomerentrennmittel und einer Base in Kontakt bringt.

**2.** Verfahren nach Anspruch 1, **dadurch gekennzeichnet, daß** es aus den folgenden Schritten besteht:

(a) Solubilisierung eines racemischen Hydantoins der Formel (II) nach Anspruch 1 in einem basischen Medium,

(b) Zugabe eines Enantiomerentrennmittels,

(c) Abtrennen des Trennmittels vom Ansatz,

(d) Hydrolyse des Ansatzes in einem basischen Medium, um das basische Salz der gewünschten chiralen Aminosäure freizusetzen,

wobei die Schritte (a) und (b) gleichzeitig oder nacheinander durchgeführt werden können.

**3.** Verfahren nach Anspruch 1 oder 2, **dadurch gekennzeichnet, daß** es mit 0,2 bis 1 Äquivalent Trennmittel in bezug auf die verwendete Menge an racemischem Hydantoin durchgeführt wird.

**4.** Verfahren nach einem der Ansprüche 1 bis 3, **dadurch gekennzeichnet, daß** es einstufig durchgeführt wird (Eintopfreaktion).

**5.** Verfahren nach einem der Ansprüche 1 bis 4, **dadurch gekennzeichnet, daß** es sich bei dem Trennmittel um ein chirales Amin oder eine chirale Säure handelt.

**6.** Verfahren nach Anspruch 5, **dadurch gekennzeichnet, daß** es sich bei dem Trennmittel um *R*-(+)-α-Methylbenzylamin oder um *S*-(-)-α-Methylbenzylamin handelt.

**7.** Herstellungsverfahren nach einem der vorhergehenden Ansprüche, **dadurch gekennzeichnet, daß** es mit 0,2 bis 0,8 Äquivalenten einer organischen oder anorganischen Base in bezug auf die verwendete Menge an racemischem Hydantoin durchgeführt wird.

**8.** Verfahren nach einem der. Ansprüche 1 bis 7, **dadurch gekennzeichnet, daß** das Hydantoin der Formel (II) in einem Wasser-Hilfslösungsmittel-System gelöst wird.

**9.** Verfahren nach Anspruch 8, **dadurch gekennzeichnet, daß** das Hilfslösungsmittel aus der Gruppe Methanol, Ethanol und Aceton stammt.

**10.** Verfahren nach Anspruch 8 oder 9, **dadurch gekennzeichnet, daß** das Verhältnis Wasser-Hilfslösungsmittel so gewählt wird, daß sich damit eine Konzentration an racemischem Hydantoin in dem Ansatz von 5 bis 30 Gew.-% erzielen läßt.

**11.** Verfahren nach einem der Ansprüche 8 bis 10, **dadurch gekennzeichnet, daß** das Wasser-Hilfslösungsmittel-Verhältnis zwischen 90:10 und 30:70 liegt.

**12.** Verfahren nach einem der vorhergehenden Ansprüche, **dadurch gekennzeichnet, daß** das Trennmittel nach der Reaktion abgetrennt wird und im wesentlichen quantitativ ohne weitere Zusatzbehandlung in einem neuen Zyklus zur Herstellung von chiraler Aminosäure direkt wiederverwendet wird.

**13.** Verfahren nach einem der vorhergehenden Ansprüche, **dadurch gekennzeichnet, daß** die chiralen Aminosäuren der Formel (I) dergestalt sind, daß
$R_1$ einen, gegebenenfalls durch 1 bis 3 $R_6$-Gruppen, wie sie in Anspruch 1 definiert sind, substituierten Arylrest bedeutet und
$R_2$ einen Alkyl- oder Halogenalkylrest mit 1 bis 6 Kohlenstoffatomen in einer linearen oder verzweigten Kette bedeutet.

**14.** Verfahren nach Anspruch 13, **dadurch gekennzeichnet, daß** die chiralen Aminosäuren der Formel (I) dergestalt sind, daß
$R_1$ einen gegebenenfalls durch eine $R_6$-Gruppe wie oben definiert substituierten Phenylrest bedeutet und
$R_2$ einen Alkylrest aus der Gruppe Methyl, Ethyl, geradkettiges oder verzweigtes Propyl, geradkettiges oder verzweigtes Butyl, geradkettiges oder verzweigtes Pentyl und geradkettiges oder verzweigtes Hexyl bedeutet.

**15.** Verfahren zur Herstellung von Verbindungen der Formel (A) nach dem folgenden Reaktionsschema

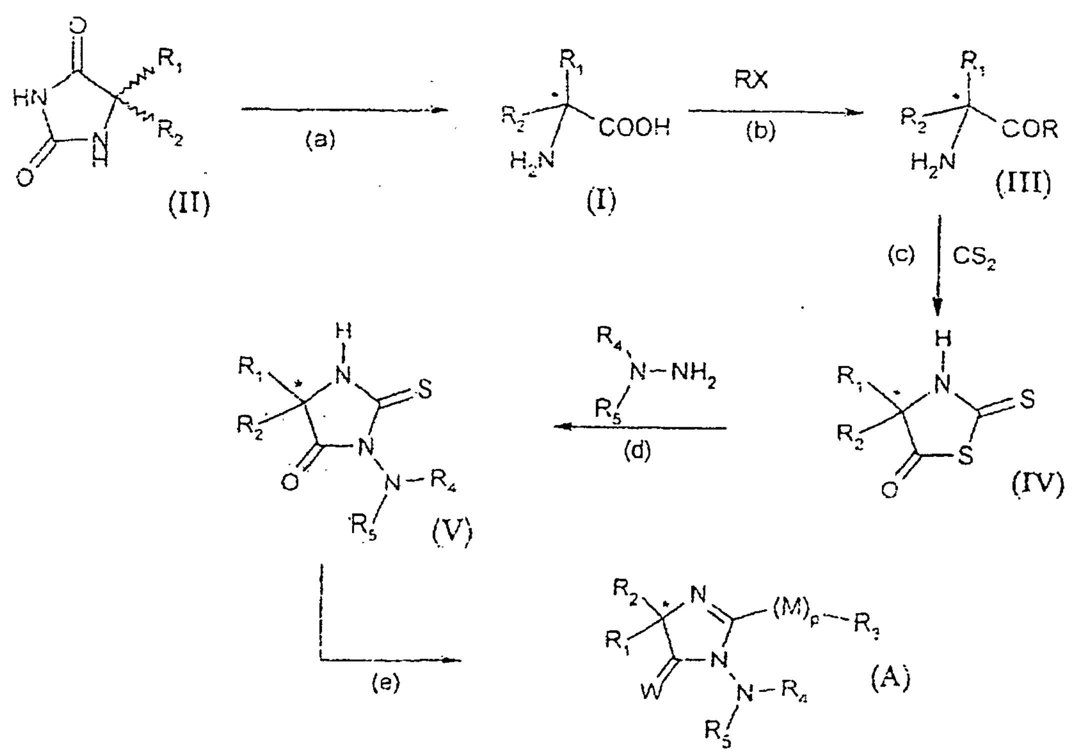

in dem
die Reste $R_1$ und $R_2$ wie in einem der vorhergehenden Ansprüche definiert sind und

- W ei.n Sauerstoff- oder Schwefelatom oder eine Gruppe S=O bedeutet,
- M ein Sauerstoff- oder Schwefelatom oder einen gegebenenfalls halogenierten $CH_2$-Rest bedeutet,
- p eine ganze Zahl gleich 0 oder 1 bedeutet,
- $R_3$
  - wenn p gleich 0 ist oder $(M)_p$ einen $CH_2$-Rest bedeutet, Wasserstoff oder einen gegebenenfalls halogenierten $C_1$-$C_2$-Alkylrest bedeutet,
  - wenn $(M)_p$ ein Sauerstoff- oder Schwefelatom bedeutet, einen gegebenenfalls halogenierten $C_1$-$C_2$-Alkylrest bedeutet,
- $R_4$
  - das Wasserstoffatom oder
  - einen Alkylrest mit 1 bis 6 Kohlenstoffatomen oder
  - einen Alkoxyalkyl-, Alkylthioalkyl-, Halogenalkyl-, Cyanoalkyl-, Thiocyanatoalkyl-, Alkenyl- oder Alkinylrest mit 2 bis 6 Kohlenstoffatomen oder
  - einens Dialkylaminoalkyl-, Alkoxycarbonylalkyloder N-Alkylcarbamoylalkylrest mit 3 bis 6 Kohlenstoffatomen oder
  - einen N,N-Dialkylcarbamoylalkylrest mit 4 bis 8 Kohlenstoffatomen oder
  - einen gegebenenfalls durch 1 bis 3 Gruppen aus der Gruppe $R_6$ substituierten Arylrest, der Phenyl, Naphthyl, Thienyl, Furyl, Pyridyl, Pyrimidyl, Pyridazinyl, Pyrazinyl, Benzothienyl, Benzofuryl, Chinolinyl, Isochinolinyl oder Methylendioxyphenyl umfaßt, oder
  - einen Arylalkyl-, Aryloxyalkyl-, Arylthioalkyloder Arylsulfonylalkylrest bedeutet,

wobei die Begriffe Aryl und Alkyl wie oben definiert sind;

- $R_5$
  - Wasserstoff oder einen Alkyl-, Halogenalkyl-, Alkylsulfonyl- oder Halogenalkylsulfonylrest mit 1 bis 6 Kohlenstoffatomen oder
  - einen Alkoxyalkyl-, Alkylthioalkyl-, Acyl-, Alkenyl-, Alkinyl-, Halogenacyl-, Alkoxycarbonyl-, Halogenalkoxycarbonyl-, Alkoxyalkylsulfonyl- oder Cyanoalkylsulfonylrest mit 2 bis 6 Kohlenstoffatomen oder
  - einen Alkoxyalkoxycarbonyl-, Alkylthioalkoxycarbonyl- oder Cyanoalkoxycarbonylrest mit 3 bis 6 Kohlenstoffatomen oder
  - den Formylrest oder einen Cycloalkyl-, Alkoxyacyl-, Alkylthioacyl-, Cyanoacyl-, Alkenylcarbonyl- oder Alkinylcarbonylrest mit 3 bis 6 Kohlenstoffatomen oder
  - einen Cycloalkylcarbonylrest mit 4 bis 8 Kohlenstoffatomen oder
  - einen Phenylrest; Arylalkylcarbonyl, insbesondere Phenylacetyl und Phenylpropionyl, Arylcarbonyl, insbesondere Benzoyl, gegebenenfalls durch 1 bis 3 Gruppen aus der Reihe $R_6$ substituiert, Thienylcarbonyl, Furylcarbonyl, Pyridylcarbonyl, Benzyloxycarbonyl, Furfuryloxycarbonyl, Tetrahydrofurfuryloxycarbonyl,

Thienylmethoxycarbonyl, Pyridylmethoxycarbonyl, Phenoxycarbonyl oder Phenylthiocarbonyl, wobei der Phenylrest selbst gegebenenfalls durch 1 bis 3 Gruppen aus der Reihe $R_6$ substituiert ist, Alkylthiocarbonyl, Halogenalkylthiocarbonyl, Alkoxyalkylthiocarbonyl, Cyanoalkylthiocarbonyl, Benzylthiocarbonyl, Furfurylthiocarbonyl, Tetrahydrofurfurylthiocarbonyl, Thienylmethylthiocarbonyl, Pyridylmethylthiocarbonyl oder Arylsulfonyl, oder

- einen gegebenenfalls durch

  - eine Alkyl- oder Halogenalkylgruppe mit 1 bis 6 Kohlenstoffatomen,
  - eine Cycloalkyl-, Alkenyl- oder Alkinylgruppe mit 3 bis 6 Kohlenstoffatomen,
  - eine Alkoxyalkyl-, Alkylthioalkyl- oder Cyanoalkylgruppe mit 2 bis 6 Kohlenstoffatomen oder
  - ein gegebenenfalls durch 1 bis 3 $R_6$-Gruppen substituiertes Phenyl

  einfach oder zweifach substituierten Carbamoylrest,

- eine gegebenenfalls durch

  - eine Alkyl- oder Halogenalkylgruppe mit 1 bis 6 Kohlenstoffatomen,
  - eine Cycloalkyl-, Alkenyl- oder Alkinylgruppe mit 3 bis 6 Kohlenstoffatomen,
  - eine Alkoxyalkyl-, Alkylthioalkyl- oder Cyanoalkylgruppe mit 2 bis 6 Kohlenstoffatomen oder
  - ein gegebenenfalls durch 1 bis 3 $R_6$-Gruppen substituiertes Phenyl

  einfach oder zweifach substituierte Sulfamoylgruppe,

  - eine Alkylthioalkylsulfonylgruppe mit 3 bis 8 Kohlenstoffatomen oder eine Cycloalkylsulfonylgruppe mit 3 bis 7 Kohlenstoffatomen
    bedeutet;

  - und $R_4$ und $R_5$ auch gemeinsam mit dem Stickstoffatom, an das sie gebunden sind, eine gegebenenfalls durch einen Alkylrest mit 1 bis 3 Kohlenstoffatomen substituierte Pyrrolidino-, Piperidino-, Morpholino- oder Piperazinogruppe bilden können;

• $R_6$

- ein Halogenatom oder

- einen Alkyl-, Halogenalkyl-, Alkoxy-, Halogenalkoxy-, Alkylthio-, Halogenalkylthio- oder Alkylsulfonylrest mit 1 bis 6 Kohlenstoffatomen oder

- einen Cycloalkyl-, Halogencycloalkyl-, Alkenyloxy-, Alkinyloxy-, Alkenylthio- oder Alkinylthiorest mit 3 bis 6 Kohlenstoffatomen oder

- die Nitro- oder Cyanogruppe oder

- einen gegebenenfalls durch einen Alkyl- oder Acylrest mit 1 bis 6 Kohlenstoffatomen oder Alkoxycarbonylrest mit 2 bis 6 Kohlenstoffatomen einfach oder zweifach substituierten Aminorest, oder

- einen Phenyl-, Phenoxy- oder Pyridyloxyrest, wobei diese Reste jeweils gegebenenfalls durch 1 bis 3 gleiche oder verschiedene Gruppen aus der Reihe $R_7$ substituiert sind,
  bedeutet;

• $R_7$

- ein Halogenatom aus der Reihe Fluor, Chlor, Brom oder Iod, oder

- einen Alkylrest mit 1 bis 6 Kohlenstoffatomen oder

- einen Alkoxy- oder Alkylthiorest mit 1 bis 6 Kohlenstoffatomen oder

- einen Halogenalkoxy- oder Halogenalkylthiorest mit 1 bis 6 Kohlenstoffatomen oder
- einen Nitril- oder Nitrorest

bedeutet;

- R einen Hydroxyrest, Alkoxy mit 1 bis 6 Kohlenstoffatomen, Benzyloxy, einen Amino-, Alkylamino- oder Dialkylaminorest oder einen Alkylaminorest mit 1 bis 6 Kohlenstoffatomen bedeutet und
- X eine Abgangsgruppe wie ein Halogenatom aus der Reihe Chlor, Brom und Iod oder einen Sulfatoder Alkylsulfonyloxy- oder Arylsulfonyloxyrest bedeutet,

wobei dieses Schema **dadurch gekennzeichnet ist, daß** es sich bei der Stufe (a) um das Verfahren zur Umwandlung von racemischen Hydantoinen der Formel (II) in Aminosäuren der Formel (I) nach einem der vorhergehenden Ansprüche handelt.

## Claims

**1.** Process for the preparation of chiral amino acids of the formula (I),

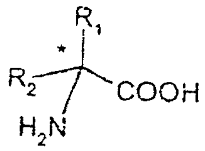

(I)

in which

- $R_1$ and $R_2$ are different and are selected from amongst
  - an alkyl or haloalkyl radical having 1 to 6 carbon atoms in a straight or branched chain,
  - an alkoxyalkyl, alkylthioalkyl, alkylsulphonylalkyl, monoalkylaminoalkyl, alkenyl or alkinyl radical having 2 to 6 carbon atoms in a straight or branched chain,
  - a dialkylaminoalkyl or cycloalkyl radical having 3 to 7 carbon atoms in a straight or branched chain,
  - an aryl radical, that is to say phenyl, naphthyl, thienyl, furyl, pyridyl, benzothienyl, benzofuryl, quinolinyl, isoquinolinyl or methylenedioxyphenyl, which is optionally substituted by 1 to 3 groups selected from amongst $R_6$, and
  - an arylalkyl, aryloxyalkyl, arylthioalkyl or arylsulphonylalkyl radical, the terms aryl and alkyl being as defined hereinabove;

    or
  - $R_1$ and $R_2$, together with the carbon to which they are linked in the cycle, can form a carbocycle or heterocycle with 5 to 7 atoms, it being possible for these cycles to be fused to a phenyl which is optionally substituted by 1 to 3 groups selected from amongst $R_6$;

- $R_6$ represents a radical selected from amongst
  - a halogen atom,
  - an alkyl, haloalkyl, alkoxy, haloalkoxy, alkylthio, haloalkylthio or alkylsulphonyl radical having 1 to 6 carbon atoms,
  - a cycloalkyl, halocycloalkyl, alkenyloxy, alkinyloxy, alkenylthio or alkinylthio radical having 3 to 6 carbon atoms,
  - the nitro or cyano group,
  - an amino radical which is optionally substituted by an alkyl or acyl radical having 1 to 6 carbon atoms or an alkoxycarbonyl radical having 2 to 6 carbon atoms, and
  - a phenyl, phenoxy or pyridyloxy radical, these radicals optionally being substituted by 1 to 3 identical or different groups selected from amongst $R_7$; and

- $R_7$ represents a radical selected from amongst

- a halogen atom selected from amongst fluorine, chlorine, bromine and iodine,
- a straight-chain or branched alkyl radical having 1 to 6 carbon atoms,
- a straight-chain or branched alkoxy or alkylthio radical having 1 to 6 carbon atoms,
- a straight-chain or branched haloalkoxy or haloalkylthio radical having 1 to 6 carbon atoms,
- a nitrile radical, and
- a nitro radical,

**characterized in that** a racemic hydantoin of the formula (II)

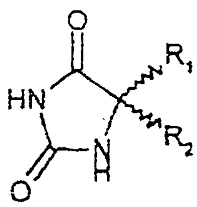

(II)

in which $R_1$ and $R_2$ are as defined for formula (I) is brought into contact with an agent for the resolution of enantiomers and with a base.

**2.** Process according to Claim 1, **characterized in that** it consists of

- (a) solubilizing a racemic hydantoin of the formula (II) according to Claim 1 in a basic medium,
- (b) adding an agent for the resolution of enantiomers,
- (c) separating the resolution agent from the reaction medium,
- (d) hydrolysing the reaction medium in a basic medium to liberate the basic salt of the desired chiral amino acid,

it being possible to carry out steps (a) and (b) simultaneously or in succession.

**3.** Process according to Claim 1 or 2, **characterized in that** it is carried out using 0.2 to 1 equivalent of resolution agent based on the amount of racemic hydantoin used.

**4.** Process according to one of Claims 1 to 3, **characterized in that** it is carried out in one step (one-pot reaction).

**5.** Process according to one of Claims 1 to 4, **characterized in that** the resolution agent is a chiral amine or chiral acid.

**6.** Process according to Claim 5, **characterized in that** the resolution agent is *R*-(+)-α-methylbenzylamine or *S*-(-)-α-methylbenzylamine.

**7.** Preparation process according to one of the preceding claims, **characterized in that** it is carried out using 0.2 to 0.8 equivalents of an organic or inorganic base with respect to the amount of racemic hydantoin used.

**8.** Process according to one of Claims 1 to 7, **characterized in that** the hydantoin of the formula (II) is dissolved in a water/cosolvent system.

**9.** Process according to Claim 8, **characterized in that** the cosolvent is selected from amongst methanol, ethanol and acetone.

**10.** Process according to Claim 8 or 9, **characterized in that** the water/cosolvent ratio is such that a racemic hydantoin concentration in the reaction medium of between 5 and 30% by weight can be obtained.

**11.** Process according to one of Claims 8 to 10, **characterized in that** the water/cosolvent ratio is between 90/10 and 30/70.

**12.** Process according to one of the preceding claims, **characterized in that** the resolution agent is separated off after the reaction and recovered essentially quantitatively for the direct reuse, without any additional treatment, in a new cycle for the preparation of chiral amino acids.

13. Process according to any of the preceding claims, **characterized in that** the chiral amino acids of the formula (I) are such that

- $R_1$ represents an aryl radical which is optionally substituted by 1 to 3 $R_6$ groups as defined in Claim 1, and
- $R_2$ represents an alkyl or haloalkyl radical having 1 to 6 carbon atoms in a straight or branched chain.

14. Process according to Claim 13, **characterized in that** the chiral amino acids of the formula (I) are such that

- $R_1$ represents a phenyl radical which is optionally substituted by an $R_6$ group as defined above, and
- $R_2$ represents an alkyl radical selected from amongst methyl, ethyl, straight-chain or branched propyl, straight-chain or branched butyl, straight-chain or branched pentyl and straight-chain or branched hexyl.

15. Process for the preparation of compounds of the formula (A) according to the following reaction scheme

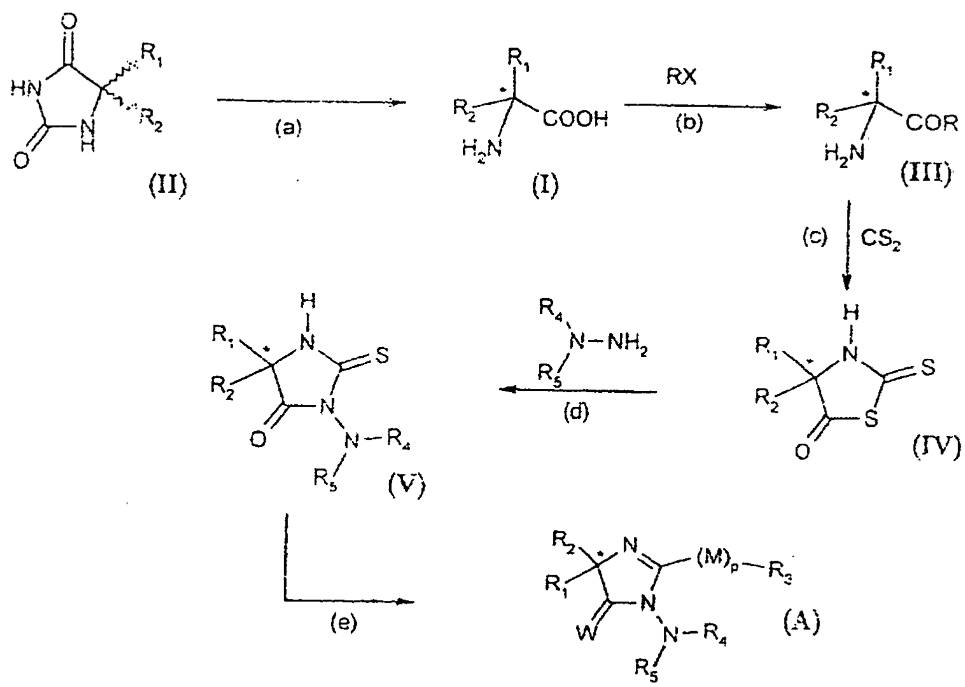

(II) (I) (III) (IV) (V) (A)

in which scheme
the radicals $R_1$ and $R_2$ are as defined in one of the preceding claims and

- W is an oxygen or sulphur atom or an S=O group,
- M represents an oxygen or sulphur atom or an optionally halogenated $CH_2$ radical,
- p is an integer of 0 or 1,
- $R_3$ represents
  - if p is 0 or $(M)_p$ is a $CH_2$ radical, hydrogen or, an optionally halogenated $C_1$-$C_2$-alkyl radical,
  - if $(M)_p$ is an oxygen or sulphur atom, an optionally halogenated $C_1$-$C_2$-alkyl radical,
- $R_4$ is
  - a hydrogen atom or
  - an alkyl radical having 1 to 6 carbon atoms or
  - an alkoxyalkyl, alkylthioalkyl, haloalkyl, cyanoalkyl, thiocyanatoalkyl, alkenyl or alkinyl radical having 2 to 6 carbon atoms or
  - a dialkylaminoalkyl, alkoxycarbonylalkyl or N-alkylcarbamoylalkyl radical having 3 to 6 carbon atoms or
  - an N,N-dialkylcarbamoylalkyl radical having 4 to 8 carbon atoms or
  - an aryl radical, which encompasses phenyl, naphthyl, thienyl, furyl, pyridyl, pyrimidyl, pyridazinyl, pyrazinyl, benzothienyl, benzofuryl, quinolinyl, isoquinolinyl or methylenedioxyphenyl, which is optionally substituted by 1 to 3 groups selected from amongst $R_6$ or
- an arylalkyl, aryloxyalkyl, arylthioalkyl or arylsulphonylalkyl radical, the terms aryl and alkyl being as defined hereinabove;

• $R_5$ is

- hydrogen or an alkyl, haloalkyl, alkylsulphonyl or haloalkylsulphonyl radical having 1 to 6 carbon atoms or
- an alkoxyalkyl, alkylthioalkyl, acyl, alkenyl, alkinyl, haloacyl, alkoxycarbonyl, haloalkoxycarbonyl, alkoxyalkylsulphonyl or cyanoalkylsulphonyl radical having 2 to 6 carbon atoms or
- an alkoxyalkoxycarbonyl, alkylthioalkoxycarbonyl or cyanoalkoxycarbonyl radical having 3 to 6 carbon atoms or
- the formyl radical or a cycloalkyl, alkoxyacyl, alkylthioacyl, cyanoacyl, alkenylcarbonyl, alkinylcarbonyl radical having 3 to 6 carbon atoms or
- a cycloalkylcarbonyl radical having 4 to 8 carbon atoms or
- a phenyl radical, arylalkylcarbonyl, in particular phenylacetyl and phenylpropionyl, arylcarbonyl, in particular benzoyl, optionally substituted by 1 to 3 groups selected from amongst $R_6$, thienylcarbonyl, furylcarbonyl, pyridylcarbonyl, benzyloxycarbonyl, furfuryloxycarbonyl, tetrahydrofurfuryloxycarbonyl, thienylmethoxycarbonyl, pyridylmethoxycarbonyl, phenoxycarbonyl or phenylthiocarbonyl, the phenyl radical itself optionally being substituted by 1 to 3 groups selected from amongst $R_6$, alkylthiocarbonyl, haloalkylthiocarbonyl, alkoxyalkylthiocarbonyl, cyanoalkylthiocarbonyl, benzylthiocarbonyl, furfurylthiocarbonyl, tetrahydrofurfurylthiocarbonyl, thienylmethylthiocarbonyl, pyridylmethylthiocarbonyl or arylsulphonyl, or
- a carbamoyl radical which is optionally mono- or disubstituted by

  - an alkyl or haloalkyl group having 1 to 6 carbon atoms,
  - a cycloalkyl, alkenyl or alkinyl group having 3 to 6 caroon atoms,
  - an alkoxyalkyl, alkylthioalkyl or cyanoalkyl group having 2 to 6 carbon atoms or
  - phenyl which is optionally substituted by 1 to 3 $R_6$ groups,

- a sulphamoyl group which is optionally mono- or disubstituted by

  - an alkyl or haloalkyl group having 1 to 6 carbon atoms,
  - a cycloalkyl, alkenyl or alkinyl group having 3 to 6 carbon atoms,
  - an alkoxyalkyl, alkylthioalkyl or cyanoalkyl group having 2 to 6 carbon atoms or
  - phenyl which is optionally substituted by 1 to 3 groups $R_6$,
  - an alkylthioalkylsulphonyl group having 3 to 8 carbon atoms or a cycloalkylsulphonyl group having 3 to 7 carbon atoms,
  - $R_4$ and $R_5$ together, along with the nitrogen atom to which they are bonded, may also form a pyrrolidino, piperidino, morpholino or piperazino group which is optionally substituted by an alkyl radical having 1 to 3 carbon atoms;

• $R_6$ is

- a halogen atom or
- an alkyl, haloalkyl, alkoxy, haloalkoxy, alkylthio, haloalkylthio or alkylsulphonyl radical having 1 to 6 carbon atoms or
- a cycloalkyl, halocycloalkyl, alkenyloxy, alkinyloxy, alkenylthic or alkinylthio radical having 3 to 6 carbon atoms or
- the nitro or cyano group or
- an amino radical which is optionally mono- or disubstituted by an alkyl or acyl radical having 1 to 6 carbon atoms or an alkoxycarbonyl radical having 2 to 6 carbon atoms,
- a phenyl, phenoxy or pyridyloxy radical, these radicals optionally being substituted by 1 to 3 identical or different groups selected from amongst $R_7$;

• $R_7$ is

- a halogen atom selected amongst fluorine, chlorine, bromine or iodine or
- an alkyl radical having 1 to 6 carbon atoms or

- an alkoxy or alkylthio radical having 1 to 6 carbon atoms or
- a haloalkoxy or haloalkylthio radical having 1 to carbon atoms or
- a nitrile or nitro radical;

• R is a hydroxy radical, an alkoxy radical having 1 to 6 carbon atoms, a benzyloxy radical, an amino, alkylamino or dialkylamino radical or an alkylamino radical having 1 to 6 carbon atoms, and
• X is a leaving group such as a halogen atom selected from amongst chlorine, bromine and iodine, or a sulphate radical, or alkylsulphonyloxy or arylsulphonyloxy,

this scheme being **characterized in that** step (a) is the process for the transformation of racemic hydantoins of the formula (II) into amino acids of the formula (I) according to one of the preceding claims.